(19) Europäisches Patentamt
European Patent Office
Office européen des brevets

(11) EP 1 898 961 B1

(12) **EUROPEAN PATENT SPECIFICATION**

(45) Date of publication and mention of the grant of the patent:
**26.02.2014 Bulletin 2014/09**

(21) Application number: **06780854.3**

(22) Date of filing: **30.06.2006**

(51) Int Cl.:
***A61K 51/00*** (2006.01) ***A61K 31/198*** (2006.01)

(86) International application number:
**PCT/JP2006/313529**

(87) International publication number:
**WO 2007/004715 (11.01.2007 Gazette 2007/02)**

# (54) METHOD TO INDIVIDUALIZE LEVODOPA/CARBIDOPA THERAPY USING A BREATH TEST

VERFAHREN ZUR INDIVIDUALISIERUNG DER LEVODOPA/CARBIDOPA-THERAPIE MIT EINEM ATEMTEST

PROCÉDÉ POUR LA PERSONNALISATION DU TRAITEMENT LÉVODOPA/CARBIDOPA AU MOYEN D'UN TEST RESPIRATOIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.06.2005 US 695503 P**

(43) Date of publication of application:
**19.03.2008 Bulletin 2008/12**

(60) Divisional application:
**13194043.9**

(73) Proprietor: **Otsuka America Pharmaceutical, Inc.**
**Rockville, MD 20850 (US)**

(72) Inventors:
- **MODAK, Anil, S.**
  **MA 01830 (US)**
- **KUROGI, Yasuhisa**
  **Princeton, NJ 08540 (US)**

(74) Representative: **Murphy, Colm Damien et al**
**Ipulse**
**26 Mallinson Road**
**London**
**SW11 1BP (GB)**

(56) References cited:
**EP-A1- 1 486 785**

- **DALY J ET AL: "Isotope studies on the mechanism of action of adrenal tyrosine hydroxylase", ARCHIVES OF BIOCHEMISTRY AND BIOPHYSICS, ACADEMIC PRESS, US, vol. 126, no. 2, 1 August 1968 (1968-08-01), pages 593-598, XP024805991, ISSN: 0003-9861, DOI: DOI:10.1016/0003-9861(68)90446-3 [retrieved on 1968-08-01]**
- **MUELLER MARTIN J ET AL: "The biosynthesis of the cularine alkaloids", LIEBIGS ANNALEN DER CHEMIE, vol. 0, no. 5, 1993, pages 557-563, XP002631496, ISSN: 0170-2041**
- **DEGRAZIA J.A. ET AL.: 'Radiorespirometry studies of the effects of L-dopa and MK486 therapy. Model application of carbon isotopes to the in vivo evaluation of intermediary metabolism during clinical drug trials' PROC. SEMIN. USE STABLE ISOTOP. CLIN. PHARMACOL. 1972, pages 71 - 98, XP003003805**
- **BRAUN U. ET AL.: 'Measurement of in vivo decarboylation of DOPA and tyrosine' CATECHOLAMINES: BASIC CLIN. FRONT., PROC. INT. CATECHOLAMINE SYMP., 4TH vol. 1, 1979, pages 880 - 882, XP003003806**
- **MENDELSON S. ET AL.: 'Decarboxylation of [1-14C]-L-dopa after intravenous and intraventricular administration to rats as measured by expired 14C-labeled carbon dioxide' JOURNAL OF PHARMACY AND PHARMACOLOGY vol. 27, no. 5, 1975, pages 372 - 374, XP003003807**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 898 961 B1

- DANIELSON T.J. ET AL.: 'Some effects of amphetamines upon the metabolism of L-phenylalanine and L-3,4-dihydroxyphenylalanine in vivo in the mouse' RESEARCH COMMUNICATIONS IN CHEMICAL PATHOLOGY AND PHARMACOLOGY vol. 51, no. 1, 1986, pages 11 - 22, XP003003808
- DURSO R. ET AL.: 'Variable absorption of carbidopa affects both peripheral and central levodopa metabolism' J. CLIN. PHARMACOL. vol. 40, no. 8, 2000, pages 854 - 860, XP003003809
- DELEU D. ET AL.: 'Clinical and pharmacokinetic comparison of oral and duodenal delivery of levodopa/carbidopa in patients with Parkinson's disease with fluctuating response to levodopa' EUR. J. CLIN. PHARMACOL. vol. 41, no. 5, 1991, pages 453 - 458, XP003003810

## Description

### FIELD OF THE INVENTION

The present invention relates, generally to a method of determining and assessing L-3,4-dihydroxyphenylalanine (a.k.a., Levodopa; L-dopa; or LD) metabolic capacity or DOPA decarboxylase (DDC) activity in an individual mammalian subject via a breath assay, by determining the relative amount of $^{13}CO_2$ exhaled by the subject upon intravenous or oral administration of a $^{13}C$-labeled substrate, such as levodopa. The present invention is useful as an *in vivo* phenotype assay for optimizing the dose and timing of administration of DOPA decarboxylase (DDC) inhibitor Carbidopa (CD) for systemic suppression of dopamine metabolism in Parkinson's disease patients, by evaluating DDC enzyme activity using the metabolite $^{13}CO_2$ in expired breath.

### BACKGROUND OF THE INVENTION

Conventional medical approaches to diagnosis and treatment of disease is based on clinical data alone, or made in conjunction with a diagnostic test(s). Such traditional practices often lead to therapeutic choices that are not optimal for the efficacy of the prescribed drug therapy or to minimize the likelihood of side effects for an individual subject. Therapy specific diagnostics (a.k.a., theranostics) is an emerging medical technology field, which provides tests useful to diagnose a disease, choose the correct treatment regime and monitor a subject's response. That is, theranostics are useful to predict and assess drug response in individual subjects, i.e., individualized medicine. For example, knowledge of a patient's phenotype or the drug metabolizing capacity can enable physicians to individualize therapy thereby avoiding potential drug related toxicity in poor metabolizers and increasing efficacy. Theranostic tests are also useful to select subjects for treatments that are particularly likely to benefit from the treatment or to provide an early and objective indication of treatment efficacy in individual subjects, so that the treatment can be altered with a minimum of delay. Theranostic tests may be developed in any suitable diagnostic testing format, which include, but is not limited to, e.g., non-invasive breath tests, immunohistochemical tests, clinical chemistry, immunoassay, cell-based technologies, and nucleic acid tests.

One conventional medical treatment in need of a reliable theranostic test is the therapeutic treatment of Parkinsons disease (hereinafter sometimes referred to as "PD") with a combination of levodopa (hereinafter sometimes referred to as "LD") and carbidopa (hereinafter sometimes referred to as "CD") (Deleu et al., Eur J Clin Pharmacol. 41: 453-8, 1991).

The symptoms of PD result largely from the loss of dopamine-producing cells in the substantia nigra region of the mammalian brain. Since dopamine does not cross the blood brain barrier $(BBB)_1$ the use of LD as a means of neurotransmitter replacement therapy in PD is now a standard clinical regimen. When LD is taken orally, some of the drug crosses the BBB into the central nervous system and is enzymatically converted to dopamine. However, LD is decarboxylated systemically in liver, kidney, the gastrointestinal tract and endothelial cells of capillary walls. This peripheral decarboxylation is responsible for significant side effects in subjects that include nausea, vomiting, cardiac arrhythmias and hypotension. In addition, any LD that is converted to dopamine systemically cannot enter the brain, resulting in diminished central nervous system dopamine level. CD is an inhibitor of aromatic amino acid decarboxylation that is useful to inhibit peripheral LD decarboxylation by DDC. Unlike LD, CD does not cross the blood-brain barrier. CD is routinely administered as a second drug with LD to inhibit peripheral decarboxylation in the treatment of PD. That is, CD prevents the breakdown of LD before it crosses into the brain.

The amount of LD entering a subject's brain is critical to optimal control of LD therapeutic levels and the consequent clinical benefit of LD. Effective administration of CD to inhibit peripheral decarboxylation of LD is an important factor affecting the amount of LD entering the brain of a subject. For example, where less CD is available or its inhibitory action compromised, peripheral metabolism of LD by DOPA decarboxylase (hereinafter sometimes referred to as "DDC") is greater and thus less LD is available for DDC-mediated enzymatic conversion in the CNS. Determination of optimal CD dosage for refinement of LD therapeutic delivery is confounded by individual subject variability in CD absorption and/or responsiveness. Studies employing stable isotope-labeled LD (Durso et al., Clin. Pharmacol., 40: 854-860, 2000) showed that absorption of CD is variable among human subjects with significant consequence to the degree of peripheral decarboxylation inhibition among subjects as well as the subsequent level of dopamine replacement in brain. Subjects can be classified as "good/rapid" CD absorbers or "poor/slow" CD absorbers (Durso et al., J. Clin, Pharmaco/., 40: 854-860, 2000). The level of DDC inhibition of individual subjects to the same dose of CD also varies. A priori there is no way of knowing whether a subject is "CD sensitive" and will respond well to CD administration or is "CD insensitive" and will not show marked inhibition of peripheral DDC with CD administration.

Mendelson et al. (J. Pharm. Pharmac., 1975, 27, 372-375) discloses a method for determining the metabolism of L-dopa in the brain and peripherally, by administering [$^{1-14}C$]L-dopa intravenously and intraventricularly to rats, and measuring the exhaled $^{14}CO_2$. A method of administering [$^{1-13}C$]L-dopa to a subject and measuring the body excretion

of either the compound itself or one of its metabolites is described in EP 1486785 A1. Daly et. al. (Arch. Biochem. Biophys., 1968, 126(2), 593-598) describe use of $^{18}$O-L-dopa in isotope studies on the mechanism of action of adrenal tyrosine hydroxylase; and Muller et. al. (Liebigs Ann. Chem., 1993, 0(5), 557-563) describe use of $^{18}$O-L-dopa in isotope studies of the biosynthesis of cularine alkaloids. Degrazia et al.( Proc. Semin. Use Stable Isotop. Clin. Pharmacol., 1972, 71-98) discloses a method for determining the optimal dose of the DOPA decarboxylase inhibitor MK486 by administering $^{14}$C-DOPA and different doses of MK486 to patients and determining the dose at which the effect of MK486 on exhaled $^{14}$CO2 reaches a plateau.

A substantial clinical concern regarding LD is its association with the development of motor complications after long-term use in subjects suffering from PD. Pulsatile dopaminergic stimulation as a result of erratic absorption and the short half-life of LD have been central issues in attempts to explain this occurrence. Evidence suggests that altering the delivery of LD to provide a more continuous supply of this drug to the brain may result in improved control of PD symptoms. Accordingly, there is a need in the art to develop new diagnostic assays useful to assess the dose dependence of CD on peripheral LD decarboxylation and LD uptake in the brain of individual subjects afflicted with PD in order to determine individual optimized LD and CD dosages.

SUMMARY OF THE INVENTION

The present invention relates to a method as defined by the claims.

In one aspect, the invention discloses a preparation for determining LD metabolic capacity or DDC enzyme activity in a mammalian subject, comprising as an active ingredient an LD in which at least one of the carbon or oxygen atoms is labeled with an isotope, wherein the preparation is capable of producing isotope-labeled $CO_2$ after administration to a mammalian subject. In one embodiment, the preparation is labeled with at least one isotope selected from $^{13}$C; $^{14}$C; and $^{18}$O.

In another aspect, the invention discloses a method for determining LD metabolic capacity, comprising administering a preparation comprising as an active ingredient an LD in which at least one of the carbon or oxygen atoms is labeled with an isotope, wherein the preparation is capable of producing isotope-labeled $CO_2$ after administration to a mammalian subject, and measuring the excretion behavior of an isotope-labeled metabolite excreted from the body. In one embodiment of the method, the isotope-labeled metabolite is excreted from the body as isotope-labeled $CO_2$ in the expired air.

In one embodiment, the method disclosed by the invention is a method for determining LD metabolic capacity in a mammalian subject, comprising administering to a mammalian subject a preparation comprising as an active ingredient an LD in which at least one of the carbon or oxygen atoms is labeled with an isotope, wherein the preparation is capable of producing isotope-labeled $CO_2$ after administration to a mammalian subject, measuring the excretion behavior of an isotope-labeled metabolite excreted from the body; and assessing the obtained excretion behavior in the subject. In one embodiment of-the method, a mammalian subject is administered a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of isotope-labeled $CO_2$ in the expired air is measured, and the obtained excretion behavior of $CO_2$ in the subject is assessed. In one embodiment of the method, a mammalian subject is administered a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of an isotope-labeled metabolite is measured, and the obtained excretion behavior in the subject or a pharmacokinetic parameter obtained therefrom is compared with the corresponding excretion behavior or parameter in a healthy subject with a normal LD metabolic capacity.

In one embodiment of the method, a mammalian subject is co-administered a DDC inhibitor CD along with a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of an isotope-labeled metabolite is measured to determine the optimal dose of CD for suppressing DCC in the subject.

In one embodiment, the method disclosed by the invention is a method for determining the existence, nonexistence, or degree of LD metabolic disorder in a mammalian subject, comprising the steps of administering to the subject, a preparation comprising as an active ingredient an LD in which at least one of the carbon or oxygen atoms is labeled with an isotope, wherein the preparation is capable of producing isotope-labeled $CO_2$ after administration to a mammalian subject, measuring the excretion behavior of an isotope-labeled metabolite excreted from the body, and assessing the obtained excretion behavior in the subject.

In one embodiment, the method disclosed by the invention is a method for determining LD metabolic capacity, comprising administering to a mammalian subject a preparation comprising as an active ingredient an LD in which at least one of the carbon or oxygen atoms is labeled with an isotope, wherein the preparation is capable of producing

isotope-labeled $CO_2$ after administration to the mammalian subject, and measuring the excretion behavior of an isotope-labeled metabolite excreted from the body. In one embodiment of the method, the isotope-labeled metabolite is excreted from the body as isotope-labeled $CO_2$ in the expired air.

In one embodiment, the method of the invention is a method for determining the efficacy of a DDC inhibitor to treat a medical condition in a first mammalian subject, comprising the steps of: (a) administering to the first subject the DDC inhibitor and an LD in which at least one of the carbon or oxygen atoms is labeled with an isotope, wherein the LD is capable of producing isotope labeled $CO_2$; (b) determining the level of LD metabolic capacity comprising the steps of measuring isotope labeled $CO_2$ produced in the first subject; and (c) comparing the level of LD metabolic capacity of the first subject to the level of a reference standard LD metabolic capacity, wherein a similarity in the level of LD metabolic capacity of the first subject compared to level of LD metabolic capacity of the reference standard indicates that the DDC inhibitor is effective to treat the medical condition in the first mammalian subject.

In one embodiment, the method disclosed by the invention is a method for determining the optimal dose a DDC inhibitor to treat a medical condition in a first mammalian subject, comprising the steps of: (a) administering to the first subject the DDC inhibitor CD and an LD in which at least one of the carbon or oxygen atoms is labeled with an isotope, wherein the LD is capable of producing isotope labeled $CO_2$; (b) determining the level of LD metabolic capacity, an excretion behavior or a pharmacokinetic parameter by measuring isotope labeled $CO_2$ produced in the subject; and (c) determining the optimal dose of CD to maximize the efficacy of LD base on the obtained results in the step (b). The optimal dose of CD when used with LD will be effectively used to treat the medical condition in the mammalian subject.

In one embodiment of the method, the level of the reference standard LD metabolic capacity is the level of LD metabolic capacity of the first subject before administration of the DDC inhibitor. In one embodiment of the method, the level of the reference standard LD metabolic capacity is the average of the level of the LD metabolic capacity of a one or more second mammalian subject.

In one embodiment, the method disclosed by the invention is a method for selecting a prophylactic or therapeutic treatment for a subject, comprising: (a) determining the phenotype, including DDC enzyme activity or LD metabolic capacity, of the subject; (b) assigning the subject to a subject class based on the phenotype of the subject; and (c) selecting a prophylactic or therapeutic treatment based on the subject class, wherein the subject class comprises two or more individuals who display a level of DDC inhibition that is at least about 10 percent lower than a reference standard level of DDC inhibition. In one embodiment of the method, comprising the following step (c) in place of the above (c); step (c) selecting a prophylactic or therapeutic treatment based on the subject class, wherein the subject class comprises two or more individuals who display a level of DDC inhibition that is at least about 10% higher than a reference standard level of DDC inhibition. In one embodiment of the method, comprising the following step (c") in place of the above (c); step (c") selecting a prophylactic or therapeutic treatment based on the subject class, wherein the subject class comprises two or more individuals who display a level of DDC inhibition within at least about 10 percent of a reference standard level of DDC inhibition. In one embodiment of the method, the treatment is selected from administering a drug, selecting a drug to be administered selecting a drug dosage, and selecting the timing of a drug administration.

In one embodiment, the method disclosed by the invention is a method for selecting a prophylactic or therapeutic treatment for a subject, comprising the steps of: (a) administering to a subject the DDC inhibitor CD and an LD in which at least one of the carbon or oxygen atoms is labeled with an isotope, wherein the LD is capable of producing isotope labeled $CO_2$; (b) determining the level of LD metabolic inhibition by CD, by measuring isotope labeled $CO_2$ produced in the subject; and (c) selecting a prophylactic or therapeutic treatment, including the timing of a CD administration, based on the level of LD metabolic inhibition by CD in the subject.

In one embodiment, the method disclosed the invention is a method for selecting a prophylactic or therapeutic treatment for a subject, comprising: (a) determining the phenotype, including DDC enzyme activity or LD metabolic activity of the subject; (b) assigning the subject to a subject class based on the phenotype of the subject; and (c) selecting a prophylactic or therapeutic treatment based on the subject class, wherein the subject class comprises two or more individuals who metabolize LD at a rate at least about 10 percent higher than a reference standard rate of LD metabolism. In one embodiment of the method, comprising the following step (c) in place of the above (c); step (c) selecting a prophylactic or therapeutic treatment based on the subject class, wherein the subject class comprises two or more individuals who metabolize LD at a rate at least about 10 percent lower than a reference standard rate of LD metabolism. In one embodiment of the method, comprising the following step (c") in place of the above (c); step (c") selecting a prophylactic or therapeutic treatment based on the subject class, wherein the subject class comprises two or more individuals who metabolize LD at a rate within at least about 10 percent of a reference standard rate of LD metabolism. In one embodiment of the method, the treatment is selected from administering a drug, selecting a drug to be administered, selecting a drug dosage, and selecting the timing of a drug administration.

In one embodiment of the method, the treatment is selected from administering a drug, selecting a drug to be administered, selecting a drug dosage, and selecting the timing of a drug administration.

In one embodiment, the method disclosed by the invention is a method for evaluating LD metabolic capacity, comprising the steps of: administering a $^{13}C$-labeled DDC substrate to a mammalian subject; measuring $^{13}CO_2$ exhaled

by the subject; and determining LD metabolic capacity from the measured $^{13}CO_2$. In one embodiment of the method, the $^{13}$C-labeled DDC substrate is a $^{13}$C-labeled LD. In one embodiment of the method, the $^{13}$C-labeled DDC substrate is administered non-invasively. In one embodiment of the method, the $^{13}$C-labeled DDC substrate is administered intravenously or orally. In one embodiment of the method, the exhaled $^{13}CO_2$ is measured spectroscopically. In one embodiment of the method, the exhaled $^{13}CO_2$ is measured by infrared spectroscopy. In one embodiment of the method, the exhaled $^{13}CO_2$ is measured with a mass analyzer. In one embodiment of the method, the exhaled $^{13}CO_2$ is measured over at least three time periods to generate a dose response curve, and the LD metabolic capacity is determined from the area under the curve. In one embodiment of the method, the exhaled $^{13}CO_2$ is measured over at least two different dosages of the $^{13}$C-labeled DDC substrate. In one embodiment of the method, the exhaled $^{13}CO_2$ is measured during at least the following time points: $t_0$, a time prior to ingesting the $^{13}$C-labeled substrate; $t_1$, a time after the $^{13}$C-labeled DDC substrate has been absorbed in the bloodstream of the subject; and $t_2$, a time during the first elimination phase. In one embodiment the method, the LD metabolic capacity is determined from as the a slope of $\delta^{13}CO_2$ at time points $t_1$ and $t_2$ calculated according to the following equation: slope = $[(\delta^{13}CO_2)_2 - (\delta^{13}CO_2)_1]/(t_2 - t_1)$- wherein $\delta^{13}CO_2$ is the amount of exhaled $^{13}CO_2$. In one embodiment of the method, an LD antagonist is administered to the subject before administrating a $^{13}$C-labeled DDC substrate.

In another aspect, the invention discloses a kit comprising: a $^{13}$C-labeled DDC substrate; and instructions provided with the substrate that describes how to determine $^{13}$C-labeled LD metabolic capacity in a subject. In one embodiment of the kit, the $^{13}$C-labeled DDC substrate is $^{13}$C-labeled LD. In one embodiment of the kit, the kit further comprises at least three breath collection bags.

In one embodiment, the kit disclosed by the invention is a kit comprising: a $^{13}$C-labeled DDC substrate and at least one least one DDC inhibitor; and instructions provided with the substrate that describe how to determine antagonist dose and timing of inhibitor dose in a subject. In one embodiment of the kit, the $^{13}$C-labeled DDC substrate is $^{13}$C-labeled LD and the inhibitor is CD. In one embodiment of the kit, the kit further comprises at least six breath collection bags.

## BRIEF DESCRIPTION OF THE DRAWINGS

The drawing figures depict preferred embodiments by way of example, not by way of limitations. In the figures, like reference numerals refer to the same or similar elements.

Figure 1 is a schematic drawing of the basal ganglia and related anatomical structures of the mammalian brain.

Figure 2 is a schematic drawing of the metabolism of levodopa.

Figure 3 is a schematic drawing of the clinical effect achieved with levodopa therapy in patients with moderate Parkinson's disease (PD). It is a citation from Obeso JA et al. (Olanow CW, Obeso JA eds. Beyond the Decade of the Brain. Vol.2. Dopamine agonists in early Parkinson's disease. Kent. UK. Wells Medical Ltd. 1997. 11-35)

Figure 4 is a graph illustrating variable carbidopa (CD) absorption in human subjects expressed as a time course of serum carbidopa (CD) concentration (ng/ml) following 50 mg oral CD administration. Durso et al. (J. Clin. Pharmacol., 40: 854-860, 2000) showed a wide variation in CD absorption. Human subjects classified as "good/rapid" CD absorbers (N=4) are designated by a solid circles. Human subjects classified as "poor/slow" CD absorbers (N=5) are designated by open circles.

Figures 5 to 8 show graphs illustrating CD-mediated suppression of peripheral LD metabolism by DDC enzyme in human subjects. Figure 5 is a graph of the presence of $^{13}CO_2$ in expired breath samples expressed as delta over baseline (DOB) of a human subject (Vlt 1) pretreated with the indicated dosage of CD as a function of time (min). Figure 6 is a graph of the percentage dose recovery (PDR) of $^{13}$C- labeled LD as $^{13}CO_2$ in expired breath samples observed in a human subject (Vlt 1) pretreated with the indicated dosage of CD as a function of time (min). Figure 7 is a graph of the presence of $^{13}CO_2$ in expired breath samples expressed as DOB of a human subject (Vlt 2) pretreated with the indicated dosage of CD as a function of time (min). Figure 8 is a graph of PDR of $^{13}$C-labeled LD as $^{13}CO_2$ in expired breath samples observed in a human subject (Vlt 2) pretreated with the indicated dosage of CD as a function of time (min).

Figures 9 to 14 show graphs illustrating the effect of timing of carbidopa (CD) dosing on peripheral LD metabolism by DDC enzyme in human subjects. Figure 9 is a graph of the time course (min) of the appearance of $^{13}CO_2$ in breath samples expressed as DOB of a human subject (Vlt 1) receiving the indicated dosage of CD either 1 h prior to LD administration or simultaneously with this LD treatment. Figure 10 is a graph of the time course (min) of the PDR of $^{13}$C-labeled LD as $^{13}CO_2$ in breath samples in a human subject (Vlt 1) administered the indicated dosage

of CD either 1 h prior to LD treatment or simultaneously with LD treatment. Figure 11 is a graph of the time course (min) of the appearance of $^{13}CO_2$ in breath samples expressed as DOB of a human subject (Vlt 2) receiving the indicated dosage of CD either 1 h prior to LD administration or simultaneously with LD treatment. Figure 12 is a graph of the time course (min) of the PDR of $^{13}C$-labeled LD as $^{13}CO_2$ in breath samples in a human subject (Vlt 2) administered the indicated dosage of CD either 1 h prior to LD treatment or simultaneously LD treatment. Figure 13 is a graph of the time course (min) of the appearance of $^{13}CO_2$ in breath samples expressed as DOB of a human subject (Vlt 2) receiving the indicated dosage of CD either 1 h prior to LD administration, 2 h prior to LD administration or simultaneously with LD treatment. Figure 14 is a graph of the time course (min) of the PDR of $^{13}C$-labeled LD as $^{13}CO_2$ in breath samples in a human subject (Vlt 2) administered the indicated dosage of CD either 1 h prior to LD treatment, 2 h prior to LD treatment, or simultaneously with LD treatment.

DETAILED DESCRIPTION OF THE INVENTION

It is to be appreciated that certain aspects, modes, embodiments, variations and features of the invention are described below in various levels of detail in order to provide a substantial understanding of the present invention. The present invention discloses a diagnostic, noninvasive, *in vivo* phenotype test to evaluate DOPA decarboxylase enzyme (DDC; EC 4.1.1.28) activity, using its enzyme substrate (e.g., levodopa, a.k.a., L-dopa or LD) labeled with isotope incorporated at least at one specific position. The present invention utilizes the DDC enzyme-substrate interaction such that there is release of stable isotope labeled $CO_2$ (e.g., $^{13}CO_2$) in the expired breath of a mammalian subject. The subsequent quantification of stable isotope labeled $CO_2$ allows for the indirect determination of pharmacokinetics of the substrate and the evaluation of DDC enzyme activity (i.e. LD metabolic capacity). In one embodiment, the invention provides a breath test for evaluation of peripheral DDC activity based on the oral or iv administration of a stable isotope $^{13}C$-labeled LD in combination with or without the DDC inhibitor carbidopa (CD; a.k.a., C-dopa), and measurement of the $^{13}CO_2/^{12}CO_2$ ratio in expired breath using commercially available instrumentation, e.g., mass or infrared (IR) spectrometers.

A substantial clinical concern regarding LD is its association with the development of motor complications after long-term use in neurotransmitter replacement therapy in subjects suffering from PD. Pulsatile dopaminergic stimulation as a result of erratic absorption and the short half-life of LD have been central issues in attempts to explain this occurrence. Evidence suggests that altering the delivery of LD to provide a more continuous supply of this drug to the brain may result in improved control of PD symptoms. The method of the invention solves a need in the art for a noninvasive method useful to define therapeutic regimens to modulate DDC activity (i.e. LD metabolic capacity) in a subject that yield more controlled and continuous LD to the brain of the subject. For example, in one embodiment, the method of the invention is useful to determine the CD dose to completely suppress peripheral DDC activity in a subject. In another embodiment, the method of the invention is also useful to determine the ideal timing of CD administration before LD dose.

The diagnostic test (breath test) of the present invention is advantageous as it is rapid and noninvasive, therefore placing less burden on the subject to give an accurate in vivo assessment of DDC enzyme activity both safely and without side effects. Accordingly, the various aspects of the present invention relate to preparations, diagnostic/theranostic methods and kits useful to identify individuals predisposed to disease or to classify individuals with regard to drug responsiveness, side effects, or optimal drug dose. Various particular embodiments that illustrate these aspects follow.

I. Definitions

As used herein, the term "clinical response" means any or all of the following: a quantitative measure of the response, no response, and adverse response (i.e., side effects).

As used herein, the term "effective amount" of a compound is a quantity sufficient to achieve a desired therapeutic and/or prophylactic effect, for example, an amount which results in the prevention of or a decrease in the symptoms associated with a disease that is being treated, e.g., PD. The amount of compound administered to the subject will depend on the type and severity of the disease and on the characteristics of the individual, such as general health, age, sex, body weight and tolerance to drugs. It will also depend on the degree, severity and type of disease.

As used herein, the term "medical condition" includes, but is not limited to, any condition or disease manifested as one or more physical and/or psychological symptoms for which treatment is desirable, and includes previously and newly identified diseases and other disorders.

As used herein, the term "subject" means that preferably the subject is a mammal, such as a human, but can also be an animal, e.g., domestic animals (e.g., dogs, cats and the like), farm animals (e.g., cows, sheep, pigs, horses and the like) and laboratory animals (e.g., monkey, rats, mice, guinea pigs and the like).

As used herein, the administration of an agent or drug to a subject includes self-administration and the administration by another. It is also to be appreciated that the various modes of treatment or prevention of medical conditions as described are intended to mean "substantial", which includes total but also less than total treatment or prevention,

and wherein some biologically or medically relevant result is achieved.

As used herein, the term "excretion behavior" includes, but is not limited to, excretion amount of a metabolite, excretion rate of a metabolite, and change in the amount and rate with the lapse of time.

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description and the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. All references cited herein are incorporated herein by reference in their entirety and for all purposes to the same extent as if each individual publication, patent, or patent application was specifically and individually indicated to be incorporated by reference in its entirety for all purposes.

II. General

L-3,4-dihydroxyphenylalanine (hereinafter, "LD"; a.k.a., (-)-L-a-amino-b-(3,4-dihydroxybenzene) propanoic acid; L-dopa; Levodopa; Dopar ®; and Larodopar®), is a naturally occurring amino acid present in both plants and animals. LD is converted into the biologically active amine, dopamine, by DDC enzyme in the animal and human body as shown below. Metabolism of LD by DDC occurs in both peripheral tissue and in the central nervous system. In the mammalian brain, the decarboxylation product dopamine occurs in high amounts in the basal ganglia.

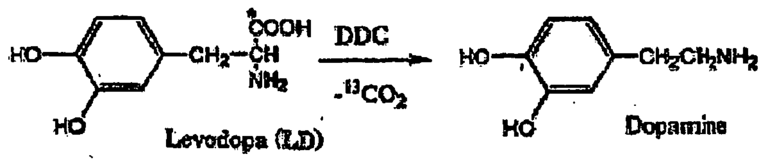


The derangement of dopamine production in the mammalian brain in conditions such as PD can lead to profound central nervous system disturbance. PD is a progressive neurodegenerative disorder that affects 1% of the population over age 65. Currently, about 1 million patients in the U.S. have PD. About 50,000 Americans are diagnosed with PD yearly according to the National Institute of Neurological Disorders and Stroke, which estimates that the total cost of health care for PD patients will exceed $5.6 billion this year.

The symptoms of PD result largely from the loss of dopamine-producing cells in the substantia nigra region of the mammalian brain (see Figure 1). Approximately 50-60% of the normal supply of dopamine has to be depleted before symptoms emerge. At first blush, neurotransmitter replacement therapy using dopamine would seem a logical approach to the management of PD symptoms, however, dopamine does not cross the BBB.

In contrast to dopamine, when LD is taken orally, some of the drug crosses the BBB into the central nervous system. Once LD crosses the BBB, it is enzymatically converted to dopamine (See Figure 2). The resulting increase in brain dopamine concentrations improves nerve conduction and alleviates the movement disorders in PD. Indeed, LD has a strong therapeutic effect in patients with PD. The use of LD as a means of neurotransmitter replacement therapy in PD is now a standard clinical regimen. Although a number of therapies have been developed in an attempt to improve PD management, e.g., dopaminergic agonists and inhibitors of COMT and MAO-B, LD remains the most effective treatment for the symptomatic control of PD.

LD is also decarboxylated systemically in liver, kidney, the gastrointestinal tract and endothelial cells of capillary walls (see Figure 2). This peripheral decarboxylation is responsible for significant side effects in subjects that include nausea, vomiting, cardiac arrhythmias and hypotension. In addition, any LD that is converted to dopamine systemically cannot enter the brain, resulting in diminished central nervous system dopamine level.

Carbidopa (hereinafter, "CD"; (-)-L-a-hydrazino-a-methyl-b-(3,4-dihydroxybenzene) propanoic acid monohydrate) is an inhibitor of aromatic amino acid decarboxylation that is useful to inhibit peripheral LD decarboxylation by DDC. Unlike LD, CD does not cross the blood-brain barrier. CD is routinely administered as a second drug with LD to inhibit peripheral decarboxylation in the treatment of PD. That is, CD prevents the breakdown of LD before it crosses into the brain.

Standard release combination formulation of CD/LD (Sinemet®) is available in 10mg/100mg, 25mg/100mg and 25mg/250mg and extended release CD/LD (Sinemet CR) in 25mg/100mg and 50mg/200mg tablets. Combination therapy of LD with CD results in less systemic LD breakdown and consequently more LD enters the brain to be converted to dopamine. The addition of CD to the PD therapeutic regimen allows administration of lower total doses of LD to a subject in need thereof. In turn, this reduces the risk of side effects from LD such as nausea and vomiting.

While LD therapy is effective in removing almost all signs and symptoms of the PD within the first three to four years; later, PD patients develop periods of time when medication does not work resulting in an "off" state. During "off spells PD patients revert to the symptoms/signs that characterize untreated PD. This problem is magnified by the fact that late-stage PD patients tend to have an "all or none" response to LD replacement therapy; that is, medication response tends to fluctuate only between good response ("on" state) and no response at all ("off" state). Consequently, in late-stage disease, when a patient is "off her symptoms/signs tend to be extremely severe. For late-stage patients with "on/off a critical amount of LD in brain (threshold level) is needed to maintain central dopamine activity at a level that results in an "on" state. If brain LD levels drop below this threshold level severe "off" time results. In addition, too much LD resulting in very high brain dopamine levels can lead to dyskinesia, hallucinations and other neurobehavioral disorders. Dyskinesia represents uncontrollable writhing movements that can become more troublesome than the PD signs themselves. This need for ideal control of LD levels is depicted in the picture below (Figure 3).

The amount of LD entering a subject's brain is critical to optimal control of LD therapeutic levels and the consequent clinical benefit of LD. Effective administration of CD to inhibit peripheral decarboxylation of LD is an important factor affecting the amount of LD entering the brain of a subject. For example, where less CD is available or its inhibitory action compromised, peripheral metabolism of LD by DDC is greater and thus less LD is available for DDC-mediated enzymatic conversion in the CNS.

Determination of optimal CD dosage for refinement of LD therapeutic delivery is confounded by individual subject variability in CD absorption and/or responsiveness. Studies employing stable isotope-labeled LD (Durso et al., J. Clin. Pharmacol., 40: 854-860, 2000) showed that absorption of CD is variable among human subjects with significant consequence to the degree of peripheral decarboxylation inhibition among subjects as well as the subsequent level of dopamine replacement in brain (See Figure 4). Subjects can be classified as "good/rapid" CD absorbers (closed circles) and "poor/slow" CD absorbers (open circles). The level of DDC inhibition of individual subjects to the same dose of CD also varies. A priori there is no way of knowing whether a subject is "CD sensitive" and will responded well to CD administration or "CD insensitive" and not show marked inhibition of peripheral DDC with CD administration. Moreover, peripheral DDC activity is not saturated at CD doses used in current clinical practice. (Durso et al., J. Clin. Pharmacol., 40: 854-860, 2000). It is important, therefore, to study the dose dependence of CD on peripheral LD decarboxylation and LD uptake in the brain of individual subjects.

PREPARATION AND METHODS

A. Isotope-labeled DDC Enzyme Substrate Preparations

The present invention discloses preparations for easily determining and assessing the DDC activity {i.e., LD metabolic capacity) in an individual mammalian subject. The preparations are useful for determining the LD metabolite behavior in a subject and easily assessing the LD metabolic capacity and identifying a clinical response and/or medical condition related to DDC activity in the subject. Specifically, the preparations of the invention are useful to determine and assess the LD metabolic capacity in an individual subject by measuring the behavior of a LD metabolite, in particular the excretion behavior of the metabolite (including excretion amount, excretion rate, and change in the amount and rate with the lapse of time), in the subject. The preparation for determining LD metabolic capacity can also be used in combination with at least one DDC enzyme inhibitor (e.g., CD) to determine the dosage of DDC inhibitor required to suppress DDC enzyme in an individual subject as well as to determine the optimal timing for dosing of DDC inhibitor in a subject.

A preparation useful in the methods of the present invention contains an isotopically labeled DDC substrate compound, such as levodopa (LD), tryptophan, phenylalanine, tyrosine, histidine, and 5-hydroxytryptophan, as an active ingredient. In one embodiment, the DDC substrate compound is an LD in which at least one of the carbon or oxygen atoms is labeled with an isotope and the preparation is capable of producing isotope labeled $CO_2$ after administration to a subject. The DDC substrate of the invention can be labeled in at least one position with $^{14}C$; $^{14}C$; and $^{18}O$. In a preferred embodiment, an LD is isotopically labeled with $^{13}C$ such that the preparation is capable of producing $^{13}CO_2$ after administration to a subject.

A preparation may be formulated with a pharmaceutically acceptable carrier. As used herein, "pharmaceutically acceptable carrier" is intended to include any and all solvents, dispersion media, coatings, antibacterial and antifungal compounds, isotonic and absorption delaying compounds, and the like, compatible with pharmaceutical administration. Suitable carriers are described in the most recent edition of Remington's Pharmaceutical Sciences (Remington: The Science And Practice Of Pharmacy, Lippincott Williams & Wilkins; 21 st Cdr edition (2005), a standard reference text in the field. Supplementary active compounds can also be incorporated into the compositions.

The method for labeling a DDC substrate with an isotope is not limited and may be a conventional method (Sasaki, "5.1 Application of Stable Isotopes in Clinical Diagnosis": Kagaku no Ryoiki (Journal of Japanese Chemistry) 107, "Application of Stable Isotopes in Medicine, Pharmacy, and Biology", pp. 149-163 (1975), Nankodo: Kajiwara,

RADIOISOTOPES, 41 , 45-48 (1992)). Some isotopically labeled DDC substrate compounds are commercially available, and these commercial products are conveniently usable. For example, $^{13}$C-labeled LD capable of producing $^{13}CO_2$ after administration to a subject is useful in the methods of the invention and is commercially available at Cambridge isotope Laboratories Inc, Andover, MA.

A pharmaceutical composition is formulated to be compatible with its intended route of administration. Examples of routes of administration include parenteral, e.g., intravenous, intradermal, subcutaneous, oral (including inhalation), transmucosal, and rectal administration. The preparation of the present invention may be in any form suitable for the purposes of the present invention. Examples of suitable forms include injections, intravenous injections, suppositories, eye drops, nasal solutions, and other parenteral forms; and solutions (including syrups), suspensions, emulsions, tablets (either uncoated or coated), capsules, pills, powders, subtle granules, granules, and other oral forms. Oral compositions generally include an inert diluent or an edible carrier.

The preparation may consist substantially of the isotope-labeled DDC substrate compound, especially LD, as an active ingredient, but may be a composition further containing a pharmaceutically acceptable carrier or additive generally used in this field according to the form of the preparation (dosage form) (composition for determining LD metabolic capacity), as long as the actions and effects of the preparation of the present invention are not impaired. In such a composition, the proportion of the isotope-labeled DDC substrate compound as an active ingredient is not limited and may be from about 0.1 wt.% to about 99 wt.% of the total dry weight of the preparation. The proportion can be suitably adjusted within the above range.

When the preparation is formed into tablets, useful carriers include, but are not limited to, e.g., lactose, sucrose, sodium chloride, glucose, urea, starches, calcium carbonate, kaolin, crystalline cellulose, silicic acid, and other excipients; simple syrups, glucose solutions, starch solutions, gelatin solutions, carboxymethyl cellulose, shellac, methyl cellulose, potassium phosphate, polyvinyl pyrrolidone, and other binders; dry starches, sodium alginate, agar powder, laminaran powder, sodium hydrogencarbonate, calcium carbonate, polyoxyethylene sorbitan, fatty acid esters, sodium lauryl sulfate, stearic acid monoglyceride, starches, lactose, and other disintegrators; sucrose, stearic acid, cacao butter, hydrogenated oils, and other disintegration inhibitors; quaternary ammonium bases, sodium lauryl sulfate, and other absorption accelerators; glycerin, starches, and other humectants; starches, lactose, kaolin, bentonite, colloidal silicic acid, and other adsorbents; and purified talc, stearate, boric acid powder, polyethylene glycol, and other lubricants. Further, the tablets may be those with ordinary coatings (such as sugar-coated tablets, gelatin-coated tablets, or film-coated tablets), double-layer tablets, or multi-layer tablets.

When forming the preparation for determining LD metabolic capacity into pills, useful carriers include, for example, glucose, lactose, starches, cacao butter, hydrogenated vegetable oils, kaolin, talc, and other excipients; gum arabic powder, tragacanth powder, gelatin, and other binders; and laminaran, agar, and other disintegrators. Capsules are prepared in a routine manner, by mixing the active ingredient according to the present invention with any of the above carriers and then filling the mixture into hardened gelatin capsules, soft capsules, or the like. Useful carriers for use in suppositories include, for example, polyethylene glycol, cacao butter, higher alcohols, esters of higher alcohols, gelatin, and semisynthetic glyceride.

When the preparation is prepared in the form of an injection, the injection solution, emulsion or suspension is sterilized and preferably isotonic with blood. Useful diluents for preparing the injection include, for example, water, ethyl alcohol, macrogol, propylene glycol, ethoxylated isostearyl alcohol, polyoxylated isostearyl alcohol, and polyoxyethylene sorbitan fatty acid esters. The injection may contain sodium chloride, glucose, or glycerin in an amount sufficient to make an isotonic solution. Also, an ordinary solubilizer, buffer, soothing agent or the like can be added to the injection.

Further, the preparation in any of the above forms may contain a pharmaceutically acceptable additive, such as a color, preservative, flavor, odor improver, taste improver, sweetener, or stabilizer. The above carriers and additives may be used either singly or in combination. The amount of the isotope-labeled DDC substrate compound, especially LD (active ingredient) per unit dose of the preparation of the present invention varies depending on the test sample and the kind of active ingredient used, and cannot be generally defined. A preferred amount is, for example, 1 to 300 mg/ body per unit dose, preferably 50 to 150 mg/body per unit dose, although it is not limited thereto as long as the above condition is satisfied.

B. Methods

A medical condition or clinical response related to DDC enzyme activity (i.e. LD metabolic capacity) in a subject can be easily assessed using the methods of the present invention by administering an isotope-labeled DDC substrate compound to the subject and measuring the excretion behavior (including excretion amount, excretion rate, and change in the amount and rate with the lapse of time) of isotope-labeled $CO_2$ in the expired air. As such, the present invention provides methods to determine the clearance of a isotope-labeled DDC substrate compound in the presence and/or absence of a DDC inhibitor to establish a more effective dosage regimen (formula, dose, number of doses, etc.) of the DDC substrate compound and/or DDC inhibitor for individual subjects based on the DDC enzyme activity in these subjects.

In one embodiment, the invention discloses a method for determining LD metabolic capacity, by administering an isotope-labeled LD preparation of the invention to a mammalian subject, and measuring the excretion behavior of an isotope-labeled metabolite excreted from the body. In one embodiment, the isotope-labeled metabolite is excreted from the body as isotope-labeled $CO_2$ (including $^{13}CO_2$, $^{14}CO_2$, and $C^{18}O_2$) in the expired air.

The isotope-labeled metabolite in the test sample can be measured and analyzed by a conventional analysis technique, such as liquid scintillation counting, mass spectroscopy, infrared spectroscopic analysis, emission spectrochemical analysis, or nuclear magnetic resonance spectral analysis, which is selected depending on whether the isotope used is radioactive or non-radioactive. The $^{13}CO_2$ can be measured by any method known in the art, such as any method that can detect the amount of exhaled $^{13}CO_2$. For example, $^{13}CO_2$ can be measured spectroscopically, such as by infrared spectroscopy. One exemplary device for measuring $^{13}CO_2$ is the UBiT.RTM.-IR300 infrared spectrometer, commercially available from Meretek (Denver, CO, USA.). The subject, having ingested the $^{13}C$-labeled DDC substrate, can exhale into a breath collection bag, which is then attached to the UBiT.RTM.-IR300. The UBiT.RTM.-IR300 measures the ratio of $^{13}CO_2$ to $^{12}CO_2$ in the breath. By comparing the results of the measurement with that of a standard, the amount of exhaled $^{13}CO_2$ can be subsequently calculated. Alternatively, the exhaled $^{13}CO_2$ can be measured with a mass analyzer.

The preparation is administered via the oral or parenteral route to a subject and an isotope-labeled metabolite excreted from the body is measured, so that the LD metabolic capacity (existence, nonexistence or degree of LD metabolic disorder (decrease/increase)) in the subject can be determined from the obtained excretion behavior (the behavior of excretion amount and excretion rate with the lapse of time) of the isotope-labeled metabolite. The metabolite excreted from the body varies depending on the kind of the active ingredient used in the preparation. For example, when the preparation comprises isotope-labeled LD as an active ingredient, the final metabolite is isotope-labeled dopamine or isotope-labeled $CO_2$. Preferably, the preparation comprises, as an active ingredient, an isotope-labeled LD that enables the excretion of isotope-labeled $CO_2$ in the expired air as a result of metabolism. Using such a preparation, the LD metabolic capacity (existence, nonexistence, or degree of LD metabolic disorder (decrease/increase)) in a subject can be determined from the excretion behavior (the behavior of excretion amount and excretion rate with the lapse of time) of isotope-labeled $CO_2$, which is obtained by administering the preparation to the subject via the oral or parenteral route and measuring isotope-labeled $CO_2$ excreted in the expired air.

In one embodiment, the invention discloses a method for determining LD metabolic capacity in a mammalian subject, by administering an isotope labeled LD preparation of the invention to a subject, measuring the excretion behavior of an isotope-labeled metabolite excreted from the body, and assessing the obtained excretion behavior in the subject. In one embodiment of the method, an isotope-labeled LD preparation is administered to a mammalian subject, the excretion behavior of isotope-labeled $CO_2$ in the expired air is measured, and assessed. In one embodiment of the method, the excretion behavior of isotope-labeled $CO_2$ or a pharmacokinetic parameter obtained therefrom is compared with the corresponding excretion behavior or parameter in a healthy subject with a normal LD metabolic capacity. That is, the LD metabolic capacity in a subject can be assessed by, for example, comparing the excretion behavior (the behavior of excretion amount or excretion rate with the lapse of time) of an isotope-labeled metabolite obtained by the above measurement, with the excretion behavior of the isotope-labeled metabolite in a reference standard, which is measured in the same manner.

Further, in place of, or in addition to, the excretion behavior of an isotope-labeled metabolite, the area under the curve (AUC), excretion rate (in particular, initial excretion rate), maximum excretion concentration ($C_{max}$), slope of the $\delta$ isotope-labeled $CO_2$ (e.g. $\delta^{13}CO_2$) as a function of time or percent dose recovery as a function of time, or a similar parameter (preferably pharmacokinetic parameter) obtained from the excretion behavior (transition curve of the excretion amount) in the subject is compared with the corresponding parameter in reference standard. In one embodiment, the reference standard is the excretion behavior observed in a one or more health subjects.

In one embodiment, LD metabolic capacity is determined by an area under the curve (AUC), which plots the amount of exhaled. Isotope-labeled $CO_2$ (e.g. $\delta^{13}CO_2$) on the y-axis versus the time after the isotope-labeled substrate is ingested. The area under the curve represents the cumulative $\delta$ isotope-labeled $CO_2$ (e.g. $\delta^{13}CO_2$) recovered.

For example, $^{13}CO_2$ is also quantified as $\delta^{13}CO_2$ (a.k.a., DOB) according to the following equation:

$\delta^{13}CO_2$ equals ($\delta^{13}CO_2$ in sample gas minus $\delta^{13}CO_2$ in baseline sample before ingestion of $^{13}C$-labeled DDC substrate) where $\delta$ values are calculated (in) by=[($R_{sample}/R_{standard}$)-1] X 1000, and "R" is the ratio of the heavy to light isotope ($^{13}CA^{12}C$) in the sample or standard.

$^{13}CO_2$ and $^{12}CO_2$ in exhaled breath samples is measured by IR spectrometry using the UBiT-IR300 (Meretek Diagnostics, Lafayette, CO; 13CO2 urea breath analyzer instruction manual. Lafayette, CO: Meretek Diagnostics; 2002; A1-A2)[12] The amount of $^{13}CO_2$ present in breath samples is expressed as delta over baseline (DOB) that represents a change in the $^{13}CO_2/^{12}CO_2$ ratio of breath samples collected before and after $^{13}C$-labeled DDC enzyme substrate ingestion, e.g., $^{13}C$-labeled LD.

$$DOB = \frac{^{13}CO_2}{^{12}CO_2}_{\text{Post dose sample}} - \frac{^{13}CO_2}{^{12}CO_2}_{\text{Pre dose sample}}$$

The amount of $^{13}C$-labeled DDC substrate absorbed and released into the breath as $^{13}CO_2$ is determined for each time point using the equation described by Amarri ( Amarri et al., Clin Nutr. 14:149-54 (1995)). These results are expressed as percentage dose recovery (PDR).

The PDR is calculated using the formula:

$$\frac{\frac{(\delta^{13}{}_{t} - \delta^{13}{}_{0}) + (\delta^{13}{}_{t+1} - \delta^{13}{}_{0})}{2} \times (t_{+1}-t) \times R_{PDB} \times 10^{-3} \times C}{\frac{\text{mg substrate}}{\text{mol. wt.}} \times \frac{P \times n}{100}} \times 100\%$$

wherein $^{13}\delta=[R_s/R_{PDB})-1] \times 10^3$

$R_s={}^{13}C$: $^{12}C$ in the sample

$R_{PDB}$ = $^{13}C$:$^{12}C$ in PDB (international standard PeeDeeBelemnite) = 0.0112372)

P is the atom % excess

n is the number of labeled carbon positions

$\delta_t$, $\delta_{t+1}$, $\delta_0$ are enrichments at times t, $t_{+1}$ and predose respectively

C is the $CO_2$ production rate (C = 300 [mmol/h]*BSA

BSA = $w^{0.5378}$* $h^{0.3963}$*0.024265 (Body Surface Area)

w: Weight (kg)

h: Height (cm)

Cmax is the highest value of DOB from the breath curve following $^{13}C$-labeled DDC substrate. In one embodiment of the method, the $^{13}C$-labeled DDC substrate is $^{13}C$-labeled LD.

As noted above, the invention provides a method for determining the existence, nonexistence, or degree of DDC metabolic disorder (i.e., a medical condition) in a mammalian subject by administering a preparation of the invention to a mammalian subject, measuring the excretion behavior of an isotope-labeled metabolite excreted from the body, and assessing the obtained excretion behavior in the subject. In a preferred embodiment of the method, the isotope-labeled metabolite is excreted from the body as isotope-labeled $CO_2$ in the expired air.

In one embodiment, the invention discloses a method for determining the efficacy of a DDC inhibitor to treat a medical condition in a mammalian subject by (a) administering to the subject the DDC inhibitor and an LD in which at least one of the carbon or oxygen atoms is labeled with an isotope, wherein the LD is capable of producing isotope labeled $CO_2$; (b) determining the level of LD metabolism by measuring isotope labeled $CO_2$ produced in the subject; and comparing the level of LD metabolism of the subject to a level of reference standard LD metabolic capacity, wherein a similarity in the level of LD metabolic capacity of the subject compared to the level of LD metabolism of the reference standard indicates that the compound is effective to treat the medical condition in the subject. In one embodiment of the method, the reference standard level of LD metabolic capacity is the level of LD metabolic capacity of the mammalian subject before administration of the DDC inhibitor. In another embodiment of the method, a level of the reference standard LD metabolic capacity is the average of the level of the LD metabolism of a one or more second mammalian subject.

In one embodiment, the invention discloses a method for selecting a prophylactic or therapeutic treatment for

a subject by (a) determining the phenotype of the subject; (b) assigning the subject to a subject class based on the phenotype of the subject; and (c) selecting a prophylactic or therapeutic treatment based on the subject class, wherein the subject class comprises two or more individuals who display a level of DDC inhibition that is at least about 10 percent lower than a reference standard level of DDC inhibition. The above-mentioned term "phenotype" includes DDC enzyme activity and LD metabolic capacity of the subject. In one embodiment of the method, the subject class comprises two or more individuals who display a level of DDC inhibition that is at least about 10% higher than a reference standard level of DDC inhibition. In one embodiment of the method, the subject class comprises two or more individuals who display a level of DDC inhibition within at least about 10 percent of a reference standard level of DDC inhibition.

In one embodiment, the invention discloses a method for selecting a prophylactic or therapeutic treatment for a subject by (a) administering to a subject the DDC inhibitor CD and an LD in which at least one of the carbon or oxygen atoms is labeled with an isotope, wherein the LD is capable of producing isotope labeled $CO_2$; (b) determining the level of LD metabolic inhibition by CD, by measuring isotope labeled $CO_2$ produced in the subject; and (c) selecting a prophylactic or therapeutic treatment, including the timing of a CD administration, based on the obtained level of LD metabolic inhibition by CD in the subject.

The therapeutic treatment selected can be administering a drug, selecting a drug dosage, and selecting the timing of a drug administration.

In one embodiment, the invention discloses a method for selecting a prophylactic or therapeutic treatment for a subject, by (a) determining the phenotype of the subject; (b) assigning the subject to a subject class based on the phenotype of the subject; and (c) selecting a prophylactic or therapeutic treatment based on the subject class, wherein the subject class comprises two or more individuals who metabolize LD at a rate at least about 10 percent higher than a reference standard rate of LD metabolism. The above-mentioned term "phenotype" includes DDC enzyme activity and LD metabolic capacity of the subject. In one embodiment of the method, the subject class comprises two or more individuals who metabolize LD at a rate at least about 10 percent lower than a reference standard rate of LD metabolism. In one embodiment of the method, the subject class comprises two or more individuals who metabolize LD at a rate within at least about 10 percent of a reference standard rate of LD metabolism. The therapeutic treatment selected can be administering a drug, selecting a drug dosage, and selecting the timing of a drug administration.

In one embodiment, the invention discloses a method for evaluating LD metabolic capacity, by administering a $^{13}C$-labeled DDC substrate compound to a mammalian subject; measuring $^{13}CO_2$ exhaled by the subject; and determining LD metabolic capacity from the measured $^{13}CO_2$. In one embodiment of the method, the $^{13}C$ -labeled DDC substrate compound is a $^{13}C$-labeled LD. In one embodiment of the method, the $^{13}C$-labeled substrate compound is administered non-invasively. In one embodiment of the method the $^{13}C$-labeled DDC substrate compound is administered intravenously or by oral route. In one embodiment of the method, the exhaled $^{13}CO_2$ is measured spectroscopically. In one embodiment of the method, the exhaled $^{13}CO_2$ is measured by infrared spectroscopy. In another embodiment of the method, the exhaled $^{13}CO_2$ is measured with a mass analyzer. In one embodiment of the method, the exhaled $^{13}CO_2$ is measured over at least three time periods to generate a dose response curve, and the LD metabolic activity is determined from the area under the curve. In one embodiment of the method, the exhaled $^{13}CO_2$ is measured over at least two different dosages of the $^{13}C$-labeled DDC substrate compound. In one embodiment of the method, the exhaled $^{13}CO_2$ is measured during at least the following time points: to, a time prior to ingesting the $^{13}C$-labeled DDC substrate compound; $t_1$, a time after the $^{13}C$-labeled DDC substrate compound has been absorbed in the bloodstream of the subject; and $t_2$, a time during the first elimination phase. In one embodiment of the method, LD metabolic capacity is determined from as the a slope of $\delta\ ^{13}CO_2$ at time points $t_1$ and $t_2$ calculated according to the following equation: slope $= [(\delta^{13}CO_2)_2-(\delta^{13}CO_2)_1]/(t_2-t_1)$- wherein $\delta\ ^{13}CO_2$ is the amount of exhaled $^{13}CO_2$. In another embodiment of the invention, a DDC inhibitor is administered to the subject before administering a $^{13}C$-labeled DDC substrate compound. In a preferred embodiment of the invention, the DDC inhibitor is carbidopa.

The method can be non-invasive, only requiring that the subject perform a breath test. The present test does not require a highly trained technician to perform the test. The test can be performed at a general practitioners office, where the analytical instrument (such as, e.g., a UBiT.RTM.-IR300) is installed. Alternatively, the test can be performed at a user's home where the home user can send breath collection bags to a reference lab for analysis.

C. Kits

The invention discloses also a kit for determining LD metabolic capacity. The kit can include $^{13}C$-labeled DDC substrate compound (e.g., $^{13}C$-labeled LD) and instructions provided with the substrate that describe how to determine LD metabolic capacity in a subject. The $^{13}C$-labeled DDC substrate compound can be supplied as a tablet, a powder or granules, a capsule, or a solution. The instructions can describe the method for LD metabolic capacity by using the area under the curve, or by the slope technique, as described above. The kit can include at least three breath collection bags.

The invention discloses also a kit comprising a $^{13}C$-labeled DDC substrate compound (e.g., $^{13}C$-labeled LD) and at least one DDC inhibitor (e.g., carbidopa); and instructions provided with the substrate that describe how to

determine determination of inhibitor dosage and timing of inhibitor dosage in a subject. The kit can include at least six breath collection bags.

The following Examples are presented in order to more fully illustrate the preferred embodiments of the invention. These Examples should in no way be construed as limiting the scope of the invention, as defined by the appended claims.

## EXAMPLES

### EXAMPLE 1 DETERMINATION OF OPTIMAL CD DOSAGE FOR SUPPRESSION OF PERIPHERAL LD METABOLISM USING THE $^{13}CO_2$ BREATH TEST METHOD OF THE INVENTION

The present studies employed the $^{13}CO_2$ breath test method of the present invention to determine the optimal dose of CD required to optimally suppress peripheral metabolism of $^{13}C$-labeled LD in individual subjects (i.e., Vlt 1 and Vlt 2). Briefly, a pre-breath test sample was collected in normal human subjects after overnight fasting (12 h) in a 1.3 L aluminum bag (Otsuka Pharmaceutical Co., Ltd., Tokyo, Japan). Briefly, normal human subjects ingested varying dosages (e.g., 25 mg-200 mg CD) 1 h prior to administration of 100 mg $^{13}C$-labeled LD on three successive days. Breath samples were collected at specified intervals following administration of $^{13}C$-labeled LD to determine peripheral metabolic decarboxylation of the drug. That is, breath samples were collected at 5 minutes intervals for 40 minutes, at 10 minutes intervals to 90 minutes and 120 minutes. Metabolism of $^{13}C$-labeled LD in the absence of CD administration served as control. The results of these studies are summarized Figures 5-8. There is a dose response to the inhibition of peripheral DDC enzyme activity by the inhibitor CD.

### EXAMPLE 2 PREADMINISTRATION OF CD YIELDS SUPERIOR SUPPRESSION OF PERIPHERAL LD DECARBOXYLATION COMPARED TO SIMULTANEOUS CD/LD ADMINISTRATION

The present studies examined the effect of CD preadministration on peripheral LD metabolism in human subjects. Briefly, normal human subjects ingested varying dosages (e.g., 25 mg-200 mg CD) either simultaneously, or up to 2 h prior to administration of $^{13}C$-labeled LD. Breath samples were collected at specified intervals following administration of $^{13}C$-labeled LD to determine peripheral metabolic decarboxylation of the $^{13}C$-labeled LD. Metabolism of $^{13}C$-labeled LD in the absence of CD administration served as control. The results of these studies are summarized below in Table 1 as well as Figures 9-14.

TABLE 1

| CD | $DOB_{20}$ | | $PDR_{40}$ | | $C_{max}$ | |
|---|---|---|---|---|---|---|
| Vlt 1 | 1h prior | simultaneous | 1h prior | simultaneous | 1h prior | simultaneous |
| 0 mg | 45.6 | 45.6 | 29.8 | 29.8 | 58.8 | 58.8 |
| 25 mg | 25.8 (43) | 35.6 (22) | 12.7 (57) | 18.7 (37) | 26.9 (54) | 47.2 (20) |
| 50 mg | 6.8 (85) | 25.5 (44) | (3.6 (88) | 11.7 (61) | 6.8 (88) | 25.5 (57) |
| 100 mg | 10.6 (77) | 16.5 (64) | 5.3 (82) | 9.1 (69) | 10.6 (82) | 17.5 (70) |
| Vlt 2 | 1h prior | simultaneous | 1h prior | simultaneous | 1h prior | simultaneous |
| 0 mg | 36.7 | 36.7 | 18.8 | 18.8 | 36.7 | 36.7 |
| 50 mg | 12.6 (66) | 15.4 (58) | 8.3 (56) | 11.0 (41) | 19.1 (48) | 25 (32) |
| 100 mg | 13.6 (63) | 26.7 (27) | 7.8 (59) | 14.1 (25) | 13.5 (63) | 26.7 (27) |
| 200 mg | 8.8 (76) | 20.5 (44) | 6.7 (70) | 12.0 (36) | 9.2 (75) | 20.6 (44) |
| $DOB_{20}$: DOB value at 20 minutes after administration of $^{13}C$-labeled LD<br>$PDR_{40}$: PDR value at 40 minutes after administration of $^{13}C$-labeled LD | | | | | | |

In Table 1, figures in parenthesis are percent inhibition of DDC by CD. The delta over baseline of $^{13}CO_2$ is designated as DOB. The percentage of LD dose recovered as $^{13}CO_2$ is designated as PDR. The peak breath concentration of $^{13}CO_2$ is designated as Cmax.

As shown in Table 1 and Figure 9 through Figure 14, the control level of peripheral metabolism of $^{13}C$-labeled LD in the normal human subjects was significantly inhibited in these individuals after administration of CD at all concentrations of CD tested. The inhibition of the peripheral metabolism of $^{13}C$-labeled LD was also greater when CD was

administered prior to $^{13}C$-labeled LD administration (either 1 h or 2 h) compared to the level of LD metabolism observed when CD and $^{13}C$-labeled LD were administered simultaneously (Figure 6, panel E and panel F). Specifically, the simultaneous ingestion of CD/LD with either the 25 mg or 50 mg dose of CD and 100 mg of LD did not completely suppress the peripheral decarboxylation of DCC. If CD was administered 1 h prior to LD dose, however, there was maximal suppression of DCC observed in human subjects (i.e., Vlt 1 and Vlt 2). There was no significant difference in the level of peripheral metabolism of $^{13}C$-labeled LD observed when CD was administered 1 h prior to LD administration versus administration of CD 2 h prior to LD administration.

EXAMPLE 3 BREATH TEST PROCEDURE

In one embodiment of the breath test procedure of the invention, $^{13}C$-labeleld LD (100 mg) is ingested by a subject after overnight fasting (8-12 h), over a time period of approximately 10-15 seconds. Breath samples are collected at 5 min time points up to 40 min and at 10 min intervals to 90 min and at 120 min after ingestion of $^{13}C$-labeled LD. The breath samples are collected by having the subject momentarily holding their breath for 3 seconds prior to exhaling into a sample collection bag. The breath samples are analyzed on a UBiT IR-300 spectrophotometer (Meretek, Denver, Colo.) to determine the $^{13}CO_2/^{12}CO_2$ ratio in expired breath, or sent to a reference lab. Optionally, varying doses (10-400 mg) of CD are orally administered to the subject (10 min-6 h) prior to administration of the $^{13}C$-labeleld LD.

## Claims

1. A method for individualizing levodopa/carbidopa therapy in a mammalian subject, comprising the steps of:

    i) measuring the isotope-labeled $CO_2$ exhaled by the subject having had administered thereto an isotope-labeled levodopa and a first dosage of carbidopa, wherein metabolism of the isotope-labeled levodopa produces isotope-labeled $CO_2$, and determining levodopa metabolic capacity from the measured isotope-labeled $CO_2$;
    ii) repeating the previous steps with at least a second different dosage of carbidopa; and
    iii) determining an effective dosage regimen of carbidopa to be administered to the subject.

2. The method according to claim 1, wherein the mammalian subject has Parkinson's disease.

3. The method according to claim 1, further compromising the step of comparing the measured isotope-labeled $CO_2$ in the subject or a pharmacokinetic parameter obtained there from with the corresponding measured isotope-labeled $CO_2$ or parameter in a healthy subject with a normal levodopa metabolic capacity.

4. The method according to claim 1, wherein the isotope-labeled levodopa is $^{13}C$-labeled levodopa.

5. The method according to claim 1, wherein the isotope-labeled levodopa is administered non-invasively.

6. The method according to claim 1, wherein the isotope-labeled levodopa is administered intravenously or orally.

7. The method according to claim 1, wherein the exhaled isotope-labeled $CO_2$ is measured spectroscopically or with a mass analyzer.

8. The method according to claim 8, wherein the exhaled isotope-labeled $CO_2$ is measured by an infrared spectroscopy.

9. The method according to claim 1, wherein the exhaled isotope-labeled $CO_2$ is measured over at least three time periods for each dosage of the carbidopa to generate a curve showing the isotope-labeled levodopa metabolized at each time, and the levodopa metabolic capacity is determined from the area under at least one of the curves.

10. The method according to claim 1, wherein the exhaled isotope-labeled $CO_2$ is measured during at least the following time points for each dosage of the carbidopa: $t_0$, a time prior to ingesting the isotope-labeled levodopa; $t_1$, a time after the isotope-labeled levodopa is expected to be at least partially absorbed into the bloodstream of the subject; and $t_2$, a time after the levodopa is expected to have begun elimination from the subject.

11. The method according to claim 3, wherein the pharmacokinetic parameter is selected from percent dose recovery, area under the curve, and delta over baseline derived from at least one breath curve plotting isotope-labeled $CO_2$ generated by the subject for a dosage of carbidopa.

12. The method according to claim 1, wherein at least one dosage of carbidopa is administered to the subject before administering the isotope-labeled levodopa.

13. The method according to claim 1, wherein determining the effective dosage regimen includes determining an optimal dosage of carbidopa to be administered to the subject, an ideal timing for administering the carbidopa to the subject, or the optimal number of doses of carbidopa to be administered to the subject.

14. The method according to claim 1, wherein the isotope is at least one isotope selected from the group consisting of: $^{13}C$; $^{14}C$; and $^{18}O$.

## Patentansprüche

1. Verfahren zur Individualisierung einer Levodopa/Carbidopa-Therapie bei einem Säugerprobanden, wobei das Verfahren die folgenden Schritte umfasst:

   i) Messen des isotopmarkierten $CO_2$, das von dem Probanden ausgeatmet wird, dem dazu ein isotopmarkiertes Levodopa und eine erste Carbidopa-Dosis verabreicht wurde, wobei der Stoffwechsel des isotopmarkierten Levodopas isotopmarkiertes $CO_2$ produziert, und Bestimmen der Levodopa-Stoffwechselkapazität aus dem gemessenen isotopmarkierten $CO_2$;
   ii) Wiederholen der vorherigen Schritte mit mindestens einer zweiten, unterschiedlichen Carbidopa-Dosis und
   iii) Bestimmen eines wirksamen Dosisregimes von Carbidopa, das dem Probanden zu verabreichen ist.

2. Verfahren nach Anspruch 1, wobei der Säugerproband an der Parkinson-Krankheit leidet.

3. Verfahren nach Anspruch 1, das weiterhin den Schritt des Vergleichens des gemessenen isotopmarkierten $CO_2$ in dem Probanden oder eines daraus erhaltenen pharmakokinetischen Parameters mit dem entsprechenden gemessenen isotopmarkierten $CO_2$ oder Parameter in einem gesunden Probanden mit einer normalen Levodopa-Stoffwechselkapazität umfasst.

4. Verfahren nach Anspruch 1, wobei das isotopmarkierte Levodopa mit $^{13}C$ markiertes Levodopa ist.

5. Verfahren nach Anspruch 1, wobei das isotopmarkierte Levodopa nichtinvasiv verabreicht wird.

6. Verfahren nach Anspruch 1, wobei das isotopmarkierte Levodopa intravenös oder oral verabreicht wird.

7. Verfahren nach Anspruch 1, wobei das ausgeatmete isotopmarkierte $CO_2$ spektroskopisch oder mit einem Massenanalysator gemessen wird.

8. Verfahren nach Anspruch 8, wobei das ausgeatmete isotopmarkierte $CO_2$ mittels einer Infrarotspektroskopie gemessen wird.

9. Verfahren nach Anspruch 1, wobei das ausgeatmete isotopmarkierte $CO_2$ über mindestens drei Zeiträume für jede Dosis des Carbidopas gemessen wird, um eine Kurve zu erstellen, die das zu jeder Zeit verstoffwechselte isotopmarkierte Levodopa zeigt, und die Levodopa-Stoffwechselkapazität aus der Fläche unter mindestens einer der Kurven bestimmt wird.

10. Verfahren nach Anspruch 1, wobei das ausgeatmete isotopmarkierte $CO_2$ während mindestens der folgenden Zeitpunkte für jede Dosis des Carbidopas gemessen wird: $t_0$, einer Zeit vor dem Aufnehmen des isotopmarkierten Levodopas; $t_1$, einer Zeit, nach der erwartet wird, dass das isotopmarkierte Levodopa zumindest zum Teil in dem Blutstrom des Probanden absorbiert wurde; und $t_2$, einer Zeit, nach der erwartet wird, dass das Levodopa mit der Ausscheidung aus dem Probanden begonnen hat.

11. Verfahren nach Anspruch 3, wobei der pharmakokinetische Parameter aus der prozentualen Dosiswiederfindung, der Fläche unter der Kurve und dem Delta im Vergleich zur Baseline ausgewählt ist, die aus mindestens einer Atemkurve abgeleitet werden, die isotopmarkiertes $CO_2$, das von dem Probanden für eine Carbidopa-Dosis erzeugt wird, graphisch darstellt.

**12.** Verfahren nach Anspruch 1, wobei mindestens eine Carbidopa-Dosis dem Probanden verabreicht wird, bevor das isotopmarkierte Levodopa verabreicht wird.

**13.** Verfahren nach Anspruch 1, wobei das Bestimmen des wirksamen Dosisregimes das Bestimmen einer optimalen Carbidopa-Dosis, die dem Probanden zu verabreichen sind, eines idealen Zeitpunkts zum Verabreichen des Carbidopas an den Probanden oder der optimalen Anzahl von Carbidopa-Dosen, die dem Probanden zu verabreichen sind, beinhaltet.

**14.** Verfahren nach Anspruch 1, wobei das Isotop mindestens ein Isotop ist, das aus der Gruppe ausgewählt ist, die aus folgenden Isotopen besteht: $^{13}C$; $^{14}C$ und $^{18}O$.

**Revendications**

**1.** Procédé pour la personnalisation de la thérapie à la lévodopa/carbidopa chez un sujet mammifère, comprenant les étapes consistant à :

i) mesurer le $CO_2$ marqué par un isotope exhalé par le sujet auquel ont été administrées une lévodopa marquée par un isotope et une première dose de carbidopa, dans lequel le métabolisme de la lévodopa marquée par un isotope produit du $CO_2$ marqué par un isotope et déterminer la capacité métabolique liée à la lévodopa à partir du $CO_2$ marqué par un isotope mesuré ;
ii) répéter les étapes précédentes avec au moins une deuxième dose différente de carbidopa ; et
iii) déterminer un schéma posologique efficace de carbidopa devant être administré au sujet.

**2.** Procédé selon la revendication 1, dans lequel le sujet mammifère a la maladie de Parkinson.

**3.** Procédé selon la revendication 1, comprenant en outre l'étape consistant à comparer le $CO_2$ marqué par un isotope mesuré chez le sujet ou un paramètre pharmacocinétique obtenu de lui avec le $CO_2$ marqué par un isotope mesuré ou paramètre correspondant chez un sujet en bonne santé avec une capacité métabolique normale liée à la lévodopa.

**4.** Procédé selon la revendication 1, dans lequel la lévodopa marquée par un isotope est la lévodopa marquée au $^{13}C$.

**5.** Procédé selon la revendication 1, dans lequel la lévodopa marquée par un isotope est administrée de manière non invasive.

**6.** Procédé selon la revendication 1, dans lequel la lévodopa marquée par un isotope est administrée par voie intraveineuse ou orale.

**7.** Procédé selon la revendication 1, dans lequel le $CO_2$ marqué par un isotope exhalé est mesuré de manière spectroscopique ou avec un analyseur de masse.

**8.** Procédé selon la revendication 8, dans lequel le $CO_2$ marqué par un isotope exhalé est mesuré par une spectroscopie infrarouge.

**9.** Procédé selon la revendication 1, dans lequel le $CO_2$ marqué par un isotope exhalé est mesuré sur au moins trois périodes de temps pour chaque dose de carbidopa pour générer une courbe représentant la lévodopa marquée par un isotope métabolisée à chaque fois, et la capacité métabolique liée à la lévodopa est déterminée d'après l'aire sous au moins une des courbes.

**10.** Procédé selon la revendication 1, dans lequel le $CO_2$ marqué par un isotope exhalé est mesuré pendant au moins les points temporels suivants pour chaque dose de carbidopa : $t_0$, un temps avant l'ingestion de la lévodopa marquée par l'isotope ; $t_1$, un temps après l'absorption au moins partielle attendue de la lévodopa marquée par un isotope dans le flux sanguin du sujet ; et $t_2$, un temps après le début attendu de l'élimination de la lévodopa chez le sujet.

**11.** Procédé selon la revendication 3, dans lequel le paramètre pharmacocinétique est sélectionné en fonction du pourcentage de récupération de la dose, l'aire sous la courbe, et la valeur delta au-dessus de la ligne de base dérivée d'au moins une courbe de respiration déterminant le $CO_2$ marqué par un isotope généré par le sujet pour une dose de carbidopa.

**12.** Procédé selon la revendication 1, dans lequel au moins une dose de carbidopa est administrée au sujet avant l'administration de la lévodopa marquée par un isotope.

**13.** Procédé selon la revendication 1, dans lequel la détermination du schéma posologique efficace comprend la détermination d'une dose optimale de carbidopa devant être administrée au sujet, d'un moment idéal pour l'administration de la carbidopa au sujet, ou du nombre optimal de doses de carbidopa devant être administrées au sujet.

**14.** Procédé selon la revendication 1, dans lequel l'isotope est au moins un isotope choisi dans le groupe comprenant : $^{13}C$ ; $^{14}C$ ; et 180.

FIGURE 1

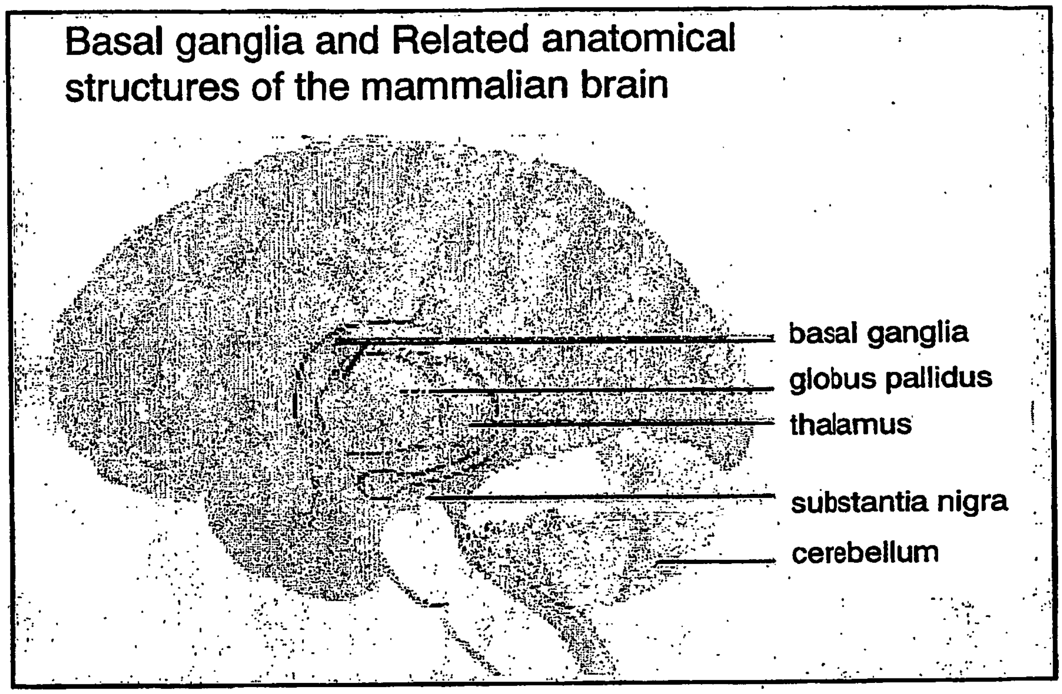
Basal ganglia and Related anatomical structures of the mammalian brain
basal ganglia
globus pallidus
thalamus
substantia nigra
cerebellum

FIGURE 2

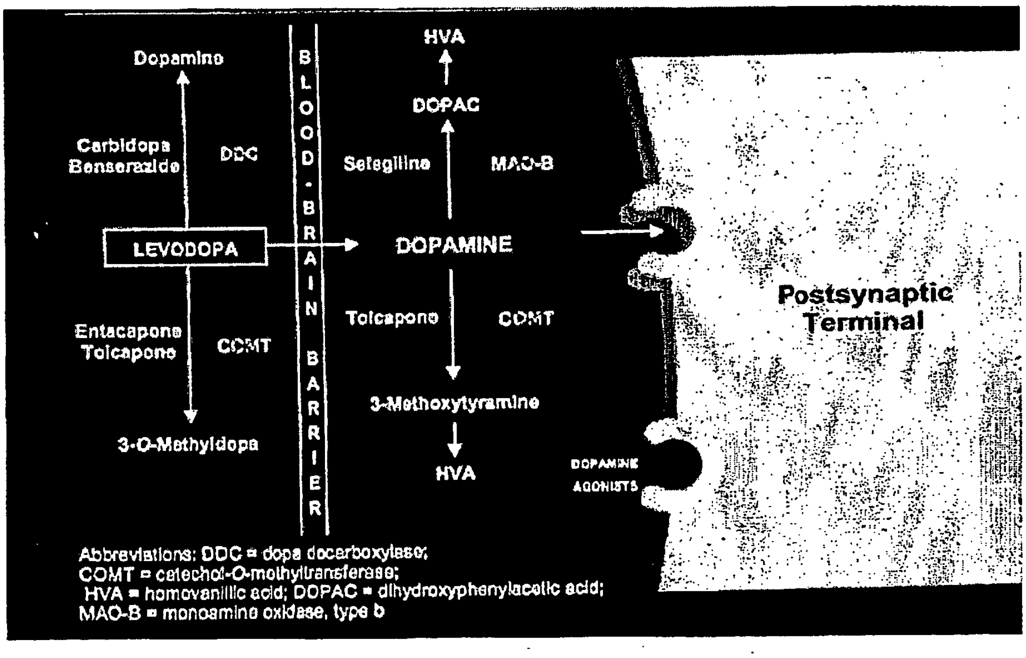
Dopamine
Carbidopa
Benserazide
DDC
LEVODOPA
Entacapone
Tolcapone
COMT
3-O-Methyldopa
BLOOD-BRAIN BARRIER
HVA
DOPAC
Selegiline
MAO-B
DOPAMINE
Tolcapone
COMT
3-Methoxytyramine
HVA
DOPAMINE AGONISTS
Postsynaptic Terminal
Abbreviations: DDC = dopa decarboxylase;
COMT = catechol-O-methyltransferase;
HVA = homovanillic acid; DOPAC = dihydroxyphenylacetic acid;
MAO-B = monoamine oxidase, type b

FIGURE 3

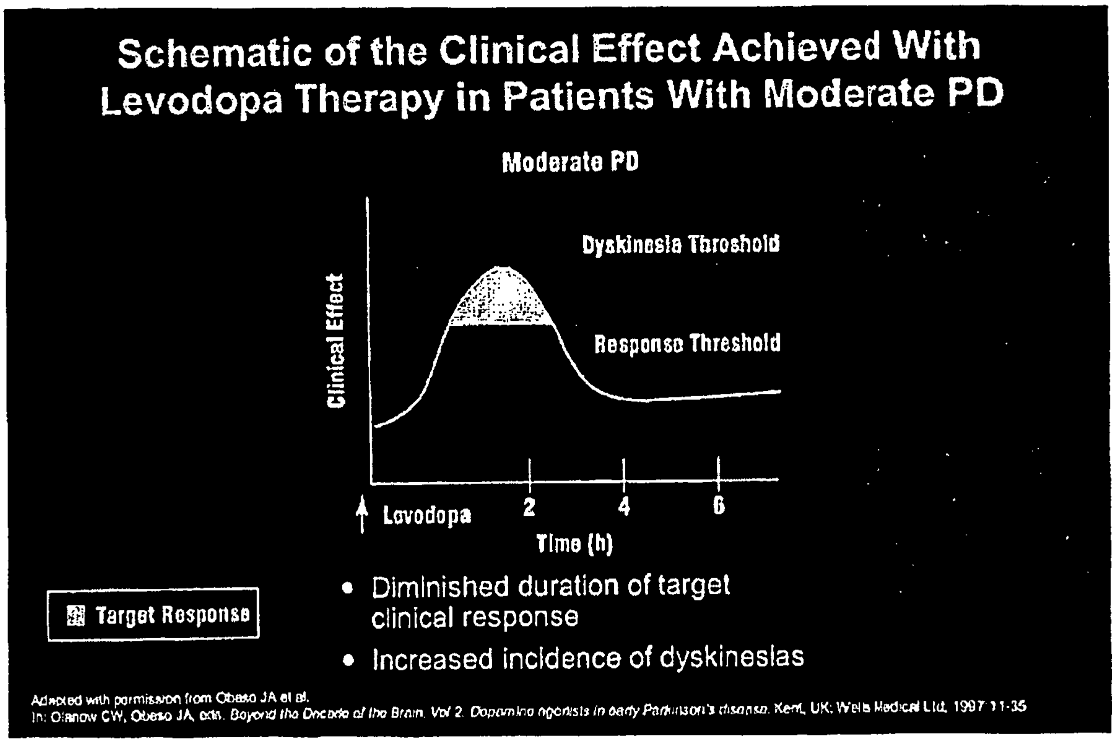
Schematic of the Clinical Effect Achieved With Levodopa Therapy in Patients With Moderate PD
Moderate PD
Dyskinesia Threshold
Response Threshold
Clinical Effect
Levodopa
2
4
6
Time (h)
Target Response
• Diminished duration of target clinical response
• Increased incidence of dyskinesias
Adapted with permission from Obeso JA et al.
In: Olanow CW, Obeso JA, eds. Beyond the Decade of the Brain. Vol 2. Dopamine agonists in early Parkinson's disease. Kent, UK: Wells Medical Ltd; 1997:11-35

FIGURE 4

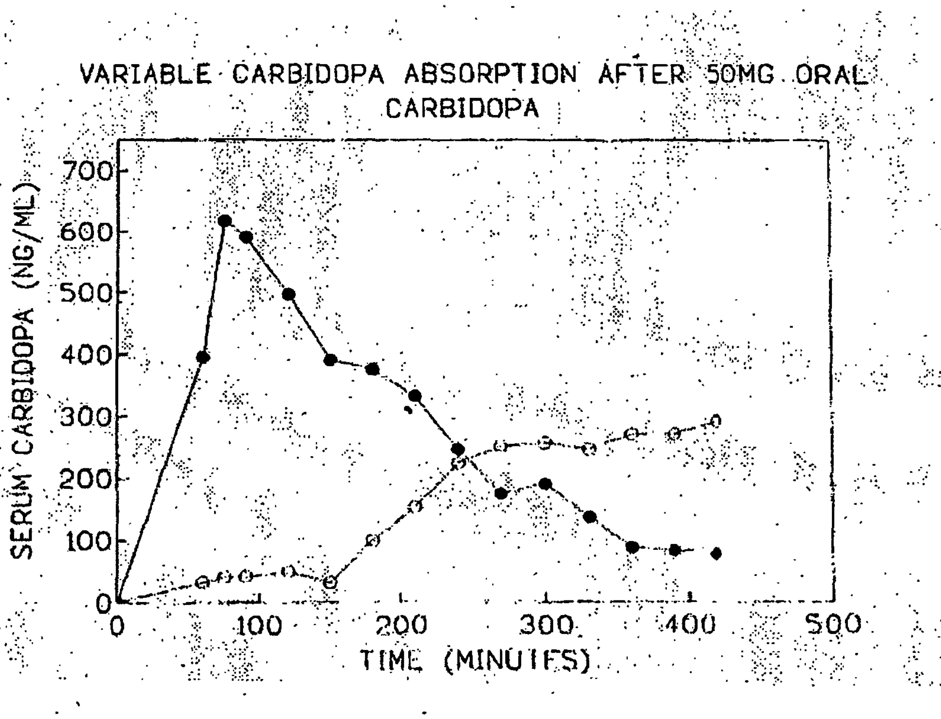
VARIABLE CARBIDOPA ABSORPTION AFTER 50MG ORAL CARBIDOPA
SERUM CARBIDOPA (NG/ML)
700
600
500
400
300
200
100
0
0
100
200
300
400
500
TIME (MINUTES)

FIGURE 5

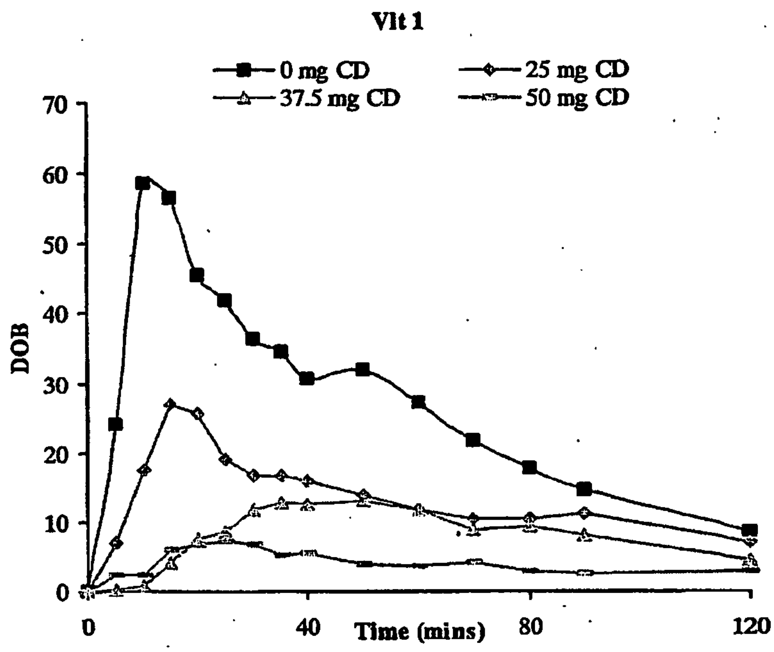


FIGURE 6

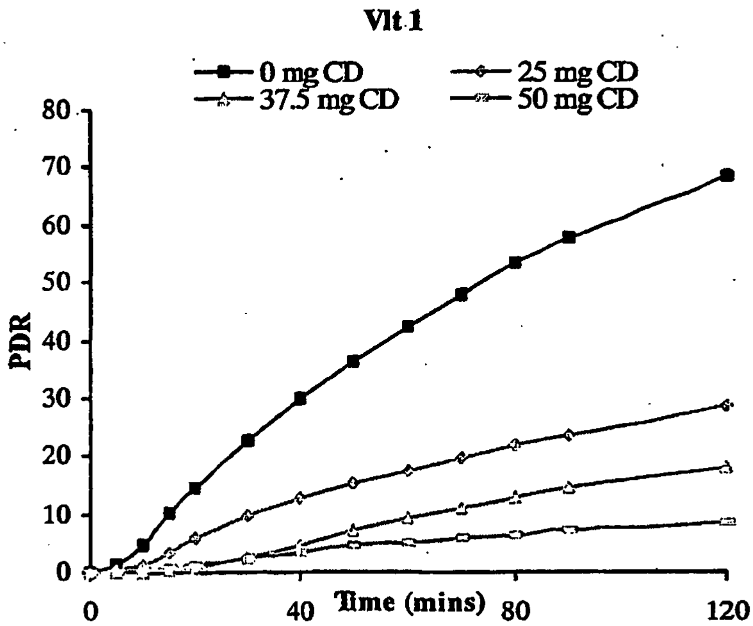

FIGURE 7

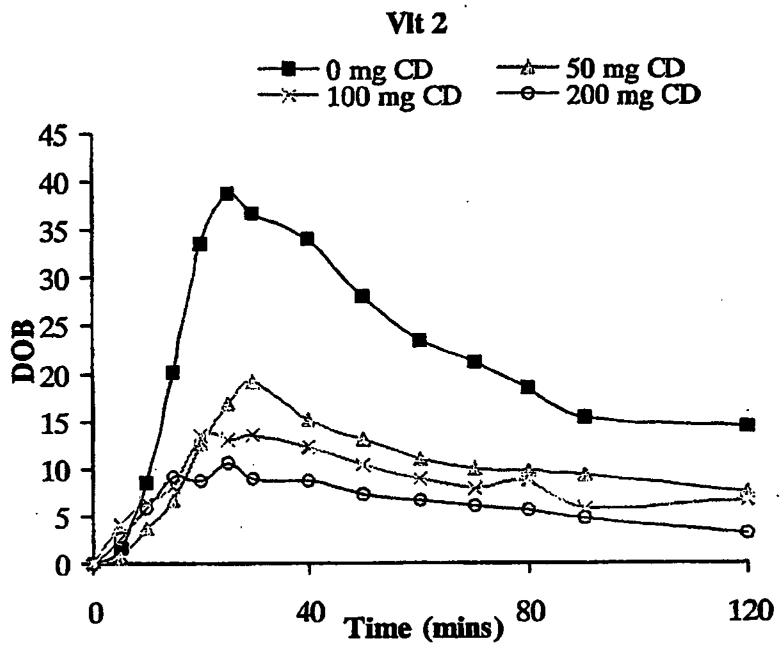


FIGURE 8

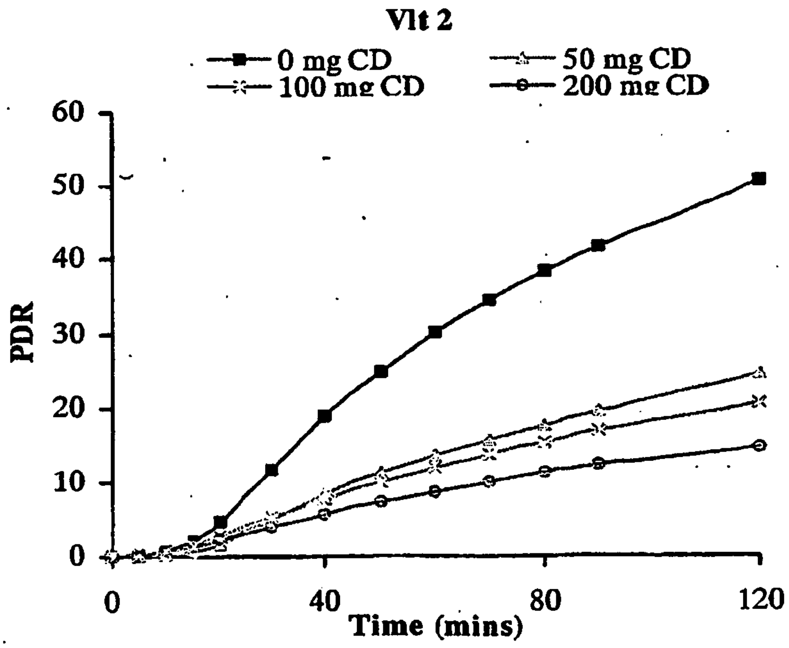

FIGURE 9

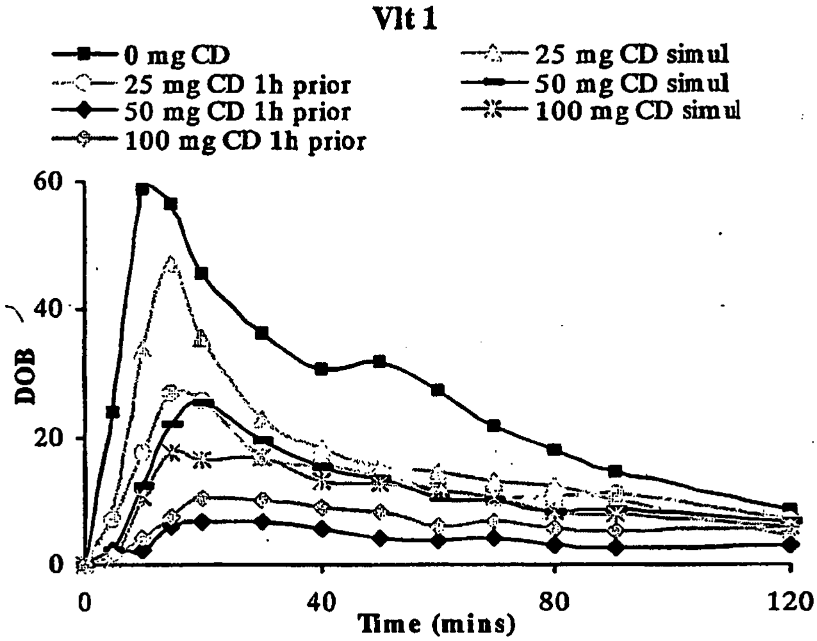


FIGURE 10

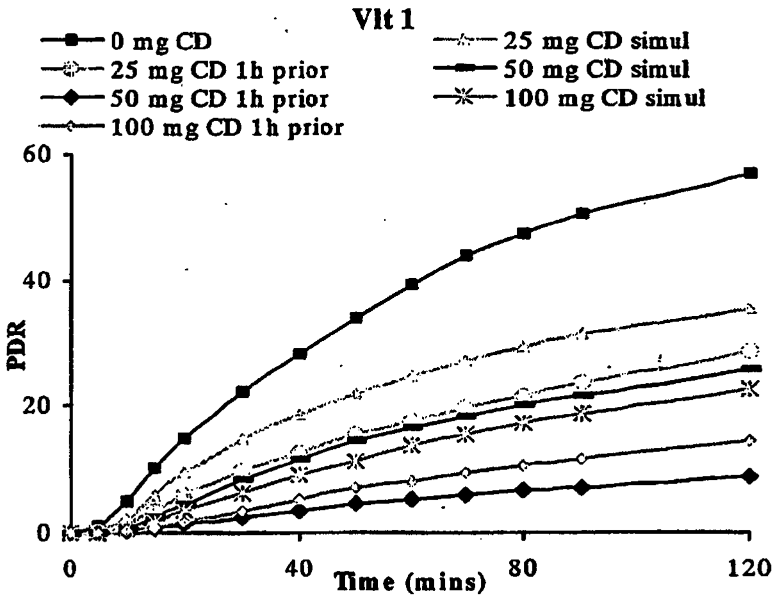

FIGURE 11

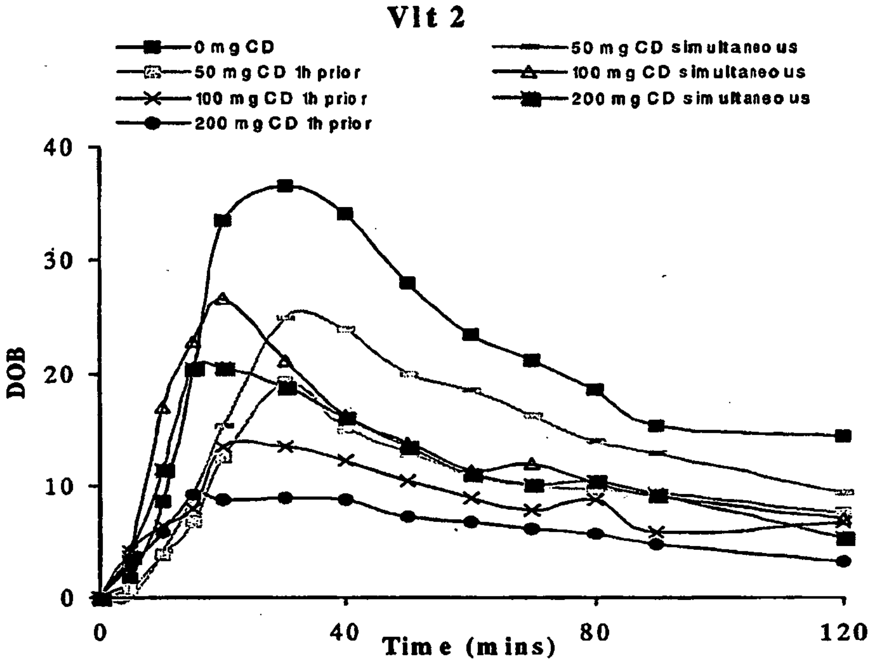


FIGURE 12

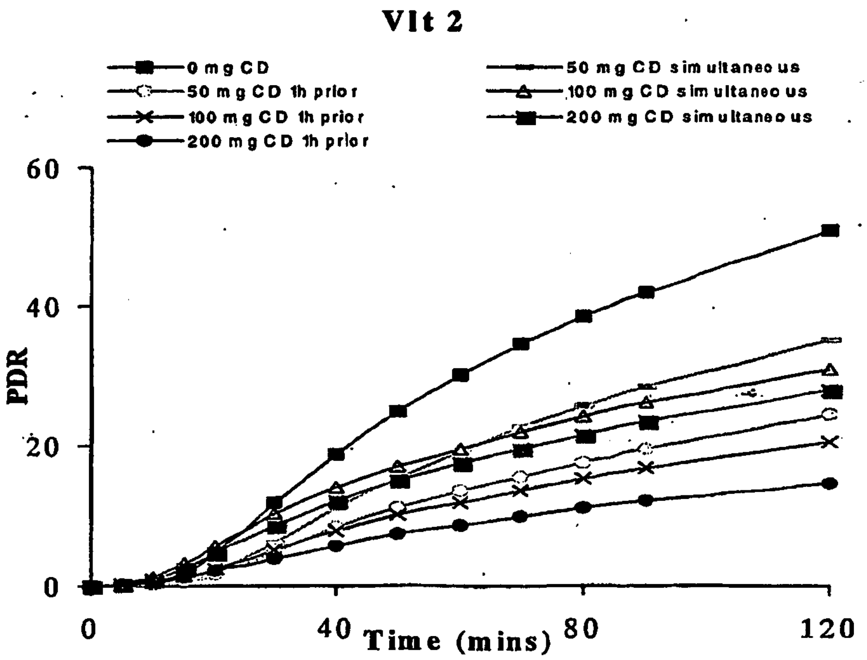

FIGURE 13

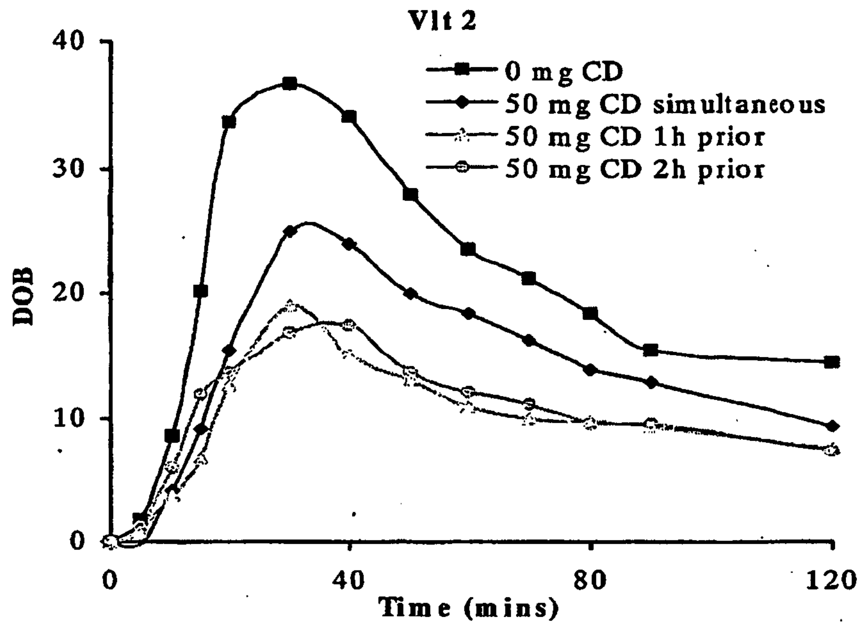


FIGURE 14

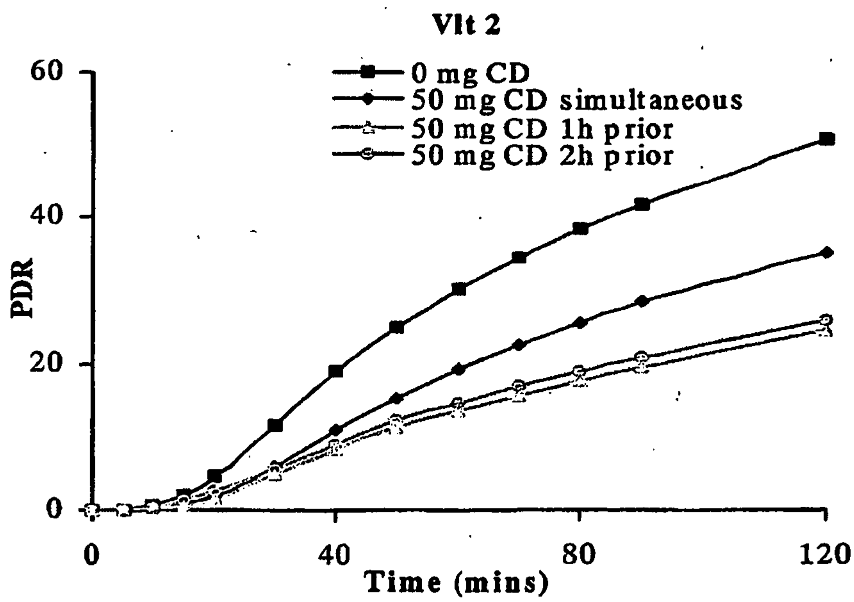

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1486785 A1

### Non-patent literature cited in the description


- **DELEU et al.** *Eur J Clin Pharmacol.,* 1991, vol. 41, 453-8
- **DURSO et al.** *Clin. Pharmacol.,* 2000, vol. 40, 854-860
- **DURSO et al.** *J. Clin, Pharmacol.,* 2000, vol. 40, 854-860
- **MENDELSON et al.** *J. Pharm. Pharmac.,* 1975, vol. 27, 372-375
- **DALY.** *Arch. Biochem. Biophys.,* 1968, vol. 126 (2), 593-598
- **MULLER.** *Liebigs Ann. Chem.,* 1993, vol. 0 (5), 557-563
- **DEGRAZIA et al.** *Proc. Semin. Use Stable Isotop. Clin. Pharmacol.,* 1972, 71-98
- **OBESO JA et al.** Beyond the Decade of the Brain. Vol.2. Dopamine agonists in early Parkinson's disease. Wells Medical Ltd, 1997, vol. 2, 11-35
- **DURSO et al.** *J. Clin. Pharmacol.,* 2000, vol. 40, 854-860
- Remington: The Science And Practice Of Pharmacy. Lippincott Williams & Wilkins, 2005
- **SASAKI.** 5.1 Application of Stable Isotopes in Clinical Diagnosis. *Kagaku no Ryoiki,* 107
- Application of Stable Isotopes in Medicine, Pharmacy, and Biology. *Nankodo: Kajiwara,* 1975, 149-163
- *RADIOISOTOPES,* 1992, vol. 41, 45-48
- Meretek Diagnostics, Lafayette, CO; CO2 urea breath analyzer instruction manual. Meretek Diagnostics. Lafayette, CO, 2002, A1-A2
- **AMARRI et al.** *Clin Nutr.,* 1995, vol. 14, 149-54